# EUROPEAN PATENT APPLICATION

(11) **EP 4 663 185 A1**
(43) Date of publication of application: **17.12.2025**
(21) Application number: 25166292.0
(22) Date of filing: 26.03.2025
(51) Int. Cl.: A61K 9/1278, A61K 9/50, A61K 47/02, A61K 31/704

(54) **A METHOD FOR ENHANCING THE EFFICIENCY OF DRUG LOADING INTO EXTRACELLULAR VESICLES AND MEDICINAL PRODUCTS THEREOF**

(30) Priority: 29.03.2024 US 202463571635 P
(71) Applicant: Shine-On BioMedical Co., Ltd., 403020 Taichung City (TW)
(72) Inventor: Ho, Hui-Chun, 116 Taipei (TW); Chen, Yi-Wen, 40763 Taichung City (TW); Kan, Kai-Wen, 407395 Taichung City (TW); Yu, Ni-Yen, 40767 Taichung City (TW); Tsai, Yi-Lun, 606 Zhongpu Township, Chiayi County (TW); Chang, Chi-Hao, Taichung City (TW); Chiang, Hung-Che, 407569 Taichung City (TW); Cho, Der-Yang, 40447 Taichung City (TW); Huang, Shi-Wei, 40447 Taichung City (TW)
(74) Representative: Cabinet Chaillot

(57) **Abstract**

A method for improving drug loading efficiency into extracellular vesicles (10), involving the preparation of drug-loaded extracellular vesicles (10) by establishing an ammonium sulfate concentration gradient using a balanced crystalloid solution. This approach facilitates the efficient encapsulation of a target drug within extracellular vesicles (10) while maintaining stability in the balanced crystalloid environment.

## Description

### FIELD OF INVENTION

The present invention relates to the field of extracellular vesicles biotechnology and the post-process, and more particularly to a method for enhancing the efficiency of drug loading into extracellular vesicles.

### BACKGROUND OF THE INVENTION

An exosome, also known as an extracellular vesicle (EV), is a microvesicle enclosed by a lipid bilayer with an average diameter of 30 to 200 nanometers (nm). This structure originates from the inward budding of the plasma membrane within parental cells, forming multivesicular bodies that are subsequently secreted into the extracellular environment. Exosomes carrying cargo can promote intercellular communication by entering recipient cells and releasing their contents.

Owing to their inherent biocompatibility and functional versatility, the exosomes have emerged as a popular tool for therapeutic interventions and diagnostic applications within biomedical industries. Through bioengineering approaches, target drugs can be encapsulated into the lumen of exosomes, enabling the targeted delivery of these drugs to specific tissues and/or cellular populations within the human body.

Despite these advancements, current methodologies for encapsulating target drugs into exosomes exhibit suboptimal efficiency. The encapsulation process often compromises the structural integrity and biological activity of the exosomes, particularly in the case of large-scale drug loading. In addition, in order to encapsulate the target drugs into exosomes, choosing a buffer for drug loading is also extremely important. However, currently used buffer (such as PBS) always led to drug precipitation and affect the loading efficiency of drugs. Therefore, there is an urgent need to develop an innovative technology that enhances drug-loading efficiency while preserving the biological activity of extracellular vesicle.

### SUMMARY OF THE INVENTION

The present invention addresses the critical need for a high-efficiency drug-loading method for extracellular vesicles that can preserve their biological activities. The disclosed method significantly enhances drug-loading efficiency through the following steps:
preparing an extracellular vesicle concentrate containing extracellular vesicles;
uniformly mixing the concentrate with an ammonium sulfate solution of an appropriate concentration, facilitating the diffusion of the ammonium sulfate solution into the lumen of the extracellular vesicle, then removing residual ammonium sulfate solution outside the lipid bilayer of the extracellular vesicle and replacing with a balanced crystalloid solution to form an extracellular vesicle reaction solution, and to establish an ammonium sulfate concentration gradient across the lipid bilayer and lumen of the extracellular vesicle; and
configuring a drug solution by dissolving the target drug in the balanced crystalloid solution, mixing the balanced crystalloid solution with the extracellular vesicle reaction solution, and then stand for reaction, when the reaction, part of the drug solution enters the lumen of the extracellular vesicle through the ammonium sulfate concentration gradient, and subsequently removing the excess drug solution, thereby completing the encapsulation of the target drug within the extracellular vesicles.

A distinctive feature of the present invention is the use of the balanced crystalloid solution as the primary medium throughout the process of ammonium sulfate gradient formation and drug loading. The balanced crystalloid solution serves as the exclusive medium for reaction and dialysis processes, including the removal of excess drug solution. This approach eliminates the need for iterative replacement of reaction solutions, thereby maintaining the stability of the extracellular vesicle's reaction environment from the initiation of the concentration gradient to the completion of drug encapsulation. This method not only enhances the quality of extracellular vesicle -based drug delivery systems, but also optimizes loading efficiency, offering significant advancements in the field. The advancement of the present invention is that the balanced crystalloid solution can mix substances from two different media without causing precipitation or destroying the original characteristics of the substances. The present invention proves that the target drug can be mixed with biological products without destroying the integrity and activity of biological membrane or lipid membrane, and the products produced using this method have demonstrated biological activity, safety and efficacy by using animal and cell experiments.

### BRIEF DESCRIPTION OF THE DRAWINGS

FIG. 1 is a schematic diagram of the drug-loading process as disclosed in the present invention;
FIG. 2 is a process flowchart outlining the manufacturing method as disclosed in the present invention;
FIG. 3 is the first experimental result obtained by the present invention;
FIG. 4 is the second experimental result obtained by the present invention;
FIG. 5 is the third experimental result obtained by the present invention;
FIG. 6 is the fourth experimental result obtained by the present invention;
FIG. 7 is the fifth experimental result obtained by the present invention;
FIG. 8A and 8B is the sixth experimental result obtained by the present invention;
FIG. 9A to 9C is the seventh experimental result obtained by the present invention; and
FIG. 10 is the eighth experimental result obtained by the present invention.

### DETAILED DESCRIPTION OF THE PREFERRED EMBODIMENTS

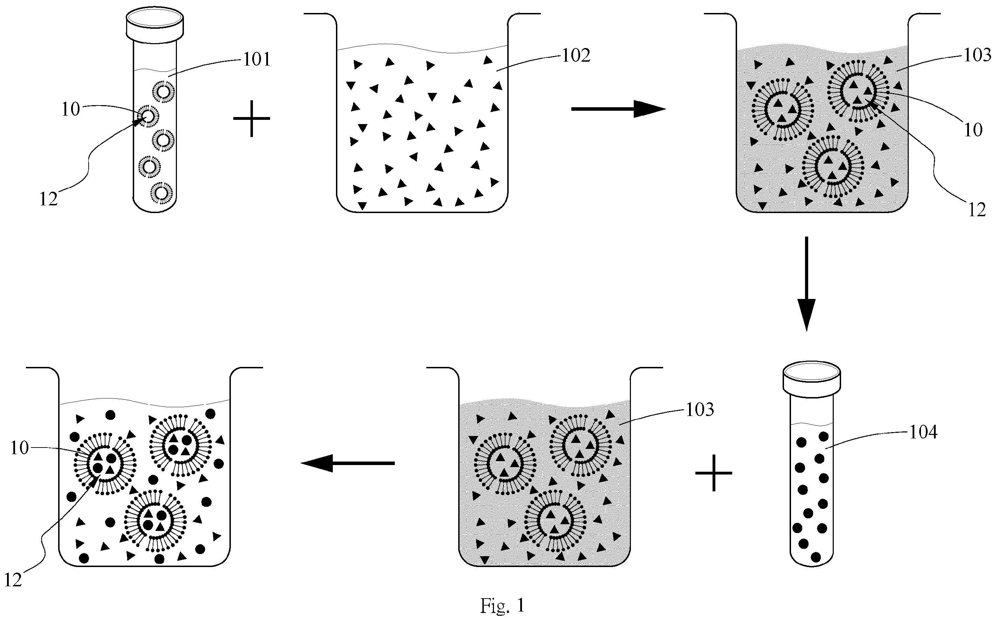

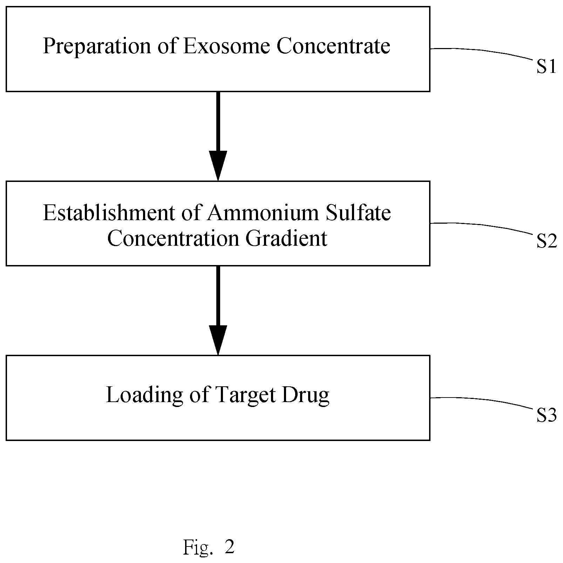

Referring to FIGS. 1 and 2, the present invention discloses a method for preparing drug-loaded extracellular vesicles, which is used to load a target drug into a lumen 12 enclosed by a lipid bilayer 11 of the extracellular vesicles, wherein the extracellular vesicles include but not limited to exosome 10, microvesicle (microparticle), apoptotic body and oncosome. In the present invention the exosome, which is smaller in size, fragile and easily broken, will be used as a subsequent illustration. The method comprises the following steps:

### Step 1: Preparation of Exosome Concentrate 101

The methods for obtaining and preparing the exosome concentrate 101 are not limited, any existing technology for obtaining the exosomes 10 may be included in the scope of the present invention.

In this embodiment, the exosomes 10 are harvested from cultures of a human embryonic kidney cell line (HEK293T). Harvesting and concentrating a culture medium of HEK293T after culturing, and forming a transitional solution after replace the medium liquid with phosphate buffered saline (PBS). Then generating an exosome concentrate 101 by further concentrating the transitional solution.

Wherein, the culture medium can be concentrated using a Tangential Flow Filtration (TFF) system.

Wherein, the particle concentration of the transitional solution is quantified using Nanoparticle Tracking Analysis (NTA), revealing a particle concentration of the exosome 10 in the range of 10⁹ to 10¹³ particles per milliliter (particles/mL).

In this process, the initial culture medium volume of 1 liter (L) is concentrated by a factor of 10 to 100. The transitional solution, with a volume of 3 to 4 milliliters (mL), is further concentrated to form the exosome concentrate 101, with the volume approximately 1 mL, by using an Amicon 3K centrifugal tube.

Furthermore, the exosomes 10 also can be bioengineered to produce a genetically modified exosome. For instance, the exosomes 10 can be genetically modified to express a target protein on the surface. This can be achieved via fusion protein technology, wherein common transmembrane proteins (like tetraspanins) within the lipid bilayer 11 of the exosome 10, such as CD63, CD81, CD82, and CD9, are fused with the specific target protein. This modification enables the exosomes to effectively carry the target protein to form the genetically modified exosome.

### Step 2: Establishment of Ammonium Sulfate Concentration Gradient

The exosome concentrate 101 is thoroughly mixed with an ammonium sulfate solution 102 at an appropriate concentration to create a homogeneous exosome mixture. Once a portion of the ammonium sulfate solution has entered the lumen 12 of the exosome 10, the remaining ammonium sulfate solution 102 outside the lipid bilayer 11 is removed and replaced with a balanced crystalloid solution, forming the exosome reaction solution. Simultaneously, an ammonium sulfate concentration gradient is established between the exterior of the lipid bilayer 11 and the lumen 12 of each the exosome 10.

Wherein, the volume ratio of the exosome concentrate 101 to the ammonium sulfate solution 102 is maintained within a range of 1:50 to 1:200.

Wherein, appropriate concentration of the ammonium sulfate solution 102 is preferably between 0.1 M to 5 M.

Wherein, the introduction of the ammonium sulfate solution into the exosome 10 is achieved via a sonication method, adding a surfactant or an extrusion method.

In the sonication method, the exosome mixture is exposed to ultrasonic energy at a power level of 20 to 80 watts for a duration of 4 minutes.

By adding the surfactant, a membrane permeability of the exosomes 10 can be increased to facilitate the entry of the ammonium sulfate solution 102 into the exosomes 10. The surfactant includes saponin or octyl phenol ethoxylate. In this embodiment, the exosome mixture reacts with the saponin with a concentration between 0.001 to 0.05%.

In the extrusion method, the exosome mixture is extruded through a polycarbonate membrane with a defined pore size between 0.1 and 0.3 µm. This process, performed iteratively using an airtight syringe, not only ensures size uniformity of the exosomes 10, but also simultaneously transfers the ammonium sulfate solution into the lumen 12, thereby establishing the ammonium sulfate concentration gradient. In some embodiment, an auto-extrusion method may be applied, replacing the airtight syringe by an extruder, which provides a stable pressure to the polycarbonate membrane. Preferably, said stable pressure is between 50-500 psi.

Removal of the residual ammonium sulfate solution 102 outside the lipid bilayer 11of the exosome 10 can be achieved by the Tangential Flow Filtration (TFF) or a size-exclusion chromatography. This step is followed by the replacement of the external medium with the balanced crystalloid solution to form the exosome reaction solution 103.

Wherein, the balanced crystalloid solution used in this process may include a Lactated Ringer's buffer or a Plasma Lyte A (PLA) solution. In the present embodiment, the Lactated Ringer's buffer will be used the following descriptions.

Furthermore, the exosome reaction solution 103 is filtered using a 0.22 µm syringe filter.

Wherein, the Nanoparticle Tracking Analysis (NTA) is used to measure the particle concentration of exosomes in the exosome reaction solution 103, with an optimal range of 10⁹ to 10¹³ particles/ml.

### Step 3: Loading of Target Drug

The target drug is dissolved in the balanced crystalloid solution, specifically Lactated Ringer's buffer, to prepare a drug solution 104. This drug solution 104 is then mixed with the exosome reaction solution 103 and left to undergo a static reaction. During the static reaction, a portion of the drug solution 104 may diffuse into the lumen 12 of the exosome 10 through the established ammonium sulfate concentration gradient. Excess drug solution that remains outside the lumen 12 of the exosome 10 is subsequently removed, completing the drug-loading process.

Wherein, a reaction amount or a reaction concentration of the target drug may depend on the selection of the target drug (such as the chemical structure, molecular weight, pharmacokinetic properties and other characteristics of the selected target drug). In the present invention, the effective concentration of the target drug in the drug solution 104 is in the range of 0.5 to 5 mg/mL, and the reaction amount of the target drug with the exosome reaction solution 103 is between 500 µg to 1000 µg.

During the reaction with the drug solution 104, the exosome reaction solution 103 contains approximately 10¹¹ exosome particles.

Wherein, the static reaction between the drug solution 104 and the exosome reaction solution 103 is carried out for a duration of 10 to 60 minutes.

Wherein, the excess drug solution 104 can be removed through the dialysis process or the size-exclusion chromatography.

In this embodiment, the dialysis process is used to remove the excess drug solution 104 which is remained outside the lumen 12 of the exosome 10. After the static reaction, the exosome reaction solution 103 is transferred into a dialysis cassette with a defined pore size and immersed in a beaker containing the balanced crystalloid solution (Lactated Ringer's buffer) for dialysis. This process leverages the size difference between the molecules of the target drug, dissolved in the drug solution 104, and the exosomes 10, allowing the free molecules of the target drug, which are smaller than the cassette's pores, remained in the excess drug solution 104 to diffuse out while retaining the larger exosomes 10, effectively removing the unencapsulated drug.

### Wherein, the dialysis process is conducted over a period of 16 to 24 hours.

The efficiency of the drug-loading process is further evaluated by analyzing the amount of drug encapsulated into the exosomes 10. This is achieved by comparing the drug-loaded exosomes 10 against a standard curve established by the target drug, enabling precise evaluation of the efficiency and quality of drug loading.

While not limited to specific compounds, the target drug preferably contains an amine group capable of forming crystalline structures with sulfate ions. This property not only helps its entry into the lumen 12 of the exosome 10 via the ammonium sulfate gradient but also enhances drug-loading efficiency through crystallization in the exosomes. In this embodiment, Doxorubicin hydrochloride (Dox-HCl) and Temozolomide (TMZ) are used respectively. To establish the standard curve of the Doxorubicin hydrochloride (Dox-HCl), a 2 mg/mL Dox-HCl solution is serially diluted, and its absorbance is measured at a wavelength of 480 nm; to establish the standard curve of the Temozolomide (TMZ), a 1 mg/mL TMZ solution is serially diluted, and its absorbance is measured at a wavelength of 330 nm.

Furthermore, an additive may be added to the exosome mixture in step 2 at the same time. The additive is not limited, and is added for the purpose of assisting in constructing the ammonium sulfate concentration gradient and promoting the drug loading effect in subsequent step 3. For example, the additive can be a pH adjuster, a catalyst, a coenzyme, a surfactant, any chemical substances or a drug. Wherein, the additive may be the saponin provided above.

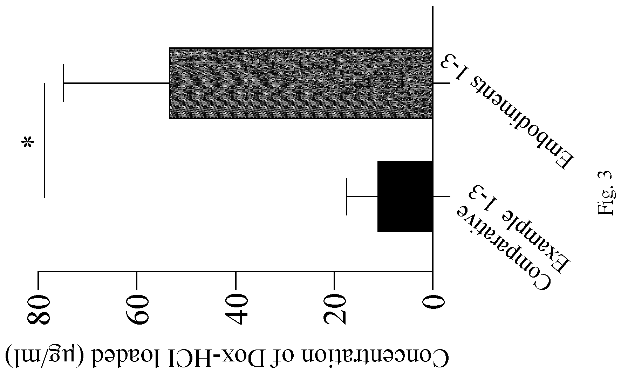

Referring to Table 1 and FIG. 3, the efficacy of Lactated Ringer's buffer as the primary medium for exosome drug loading was evaluated. The study compared the performance of the exosomes 10 and the target drugs prepared in a standard preservation solution. The difference between Embodiments 1-3 and Comparative Examples 1-3 lies in the particle concentration of the exosomes 10 within the exosome reaction solution, while the reaction amount of the exosomes 10 and the reaction amount of the target drug were maintained constant across embodiment 1-3 and comparative example 1-3.

Wherein, the preservation solution is a mixture including sucrose, polysorbate 80 and sodium acetate, the ingredients above are commonly used to preserve and stabilize drugs and protein-based solution.

**Table 1**

| | **Buffer Used in Drug Loading** | **Exosome Particle Concentration (particles/mL)** | **Target Drug Reaction Amount (µg)** | **Concentration of target drug loaded (µg/mL)** |
|---|---|---|---|---|
| **Embodiment 1** | Lactated Ringer's Buffer | 3.3E11 | 500 | 46.1 |
| **Comparative Example 1** | Preservation Solution | 2.4E11 | 500 | 7.0 |
| **Embodiment 2** | Lactated Ringer's Buffer | 8E11 | 500 | 38.1 |
| **Comparative Example 2** | Preservation Solution | 7.1E11 | 500 | 9.3 |
| **Embodiment 3** | Lactated Ringer's Buffer | 3.3E11 | 500 | 77.8 |
| **Comparative Example 3** | Preservation Solution | 3.3E11 | 500 | 18.4 |

As shown in FIG. 3, paired sample t-tests were conducted between each embodiment and the corresponding comparative example. The results revealed that Embodiments 1-3 exhibited superior drug-loading efficiencies compared to Comparative Examples 1-3, achieving approximately fourfold increases in efficiency. This demonstrates the significant enhancement in drug-loading efficacy provided by using the Lactated Ringer's buffer as the reaction medium for exosomes 10.

Referring to Table 2, in order to verify whether the establishment of the ammonium sulfate concentration gradient has an impact on the drug loading of the exosomes 10, the drug loading effect of exosomes 10 are be compared between Experiment 1 and Experiment 1A, which are established the ammonium sulfate concentration gradient through incubation and sonication respectively. The ammonium sulfate solution 102 in Experiment 1 and Experiment 1A was made from lactated Ringer's solution.

In the Experiment 1, the exosome concentrate 101 and the ammonium sulfate solution 102 were mixed and the ammonium sulfate concentration gradient of the exosome is established by using the sonication method, then the exosome is allowed to react with the target drug (Dox-HCl). In contrary, the ammonium sulfate concentration gradient of the exosome in the Experiment 1A is established through incubation that the ammonium sulfate solution 102 may passively loaded into the lumen 12 of the exosomes 10. In a control group, the exosome concentrate 101 is mixed with the PBS and allowed to stand for incubation as a comparison. Comparing with the control group, the exosomes 10 indeed exhibit the loading effect of the target drug after establishing an ammonium sulfate concentration gradient in the experiment 1A. Comparing the results of the three groups in table 2, it can be found that the exosomes 10 significantly improve the loading effect of the target drug and optimize the overall drug loading capacity, according to the concentration of target drug after the drug loading process, by establishing the ammonium sulfate concentration gradient through the sonication method.

**Table 2**

| | **Exosome Particle Concentration (particles/mL)** | **Ammoniu m sulfate solution** | **Method for establish the ammonium sulfate concentratio n gradient** | **Target Drug Reaction Amount (µg)** | **Concentration of target drug loaded (µg/mL)** |
|---|---|---|---|---|---|
| **Experiment 1** | 3.3E11 | Yes | Sonication | 500 | 77.8 |
| **Experiment 1A** | 4.4E11 | Yes | Incubation | 500 | 73.6 |
| **Control** | 3.9E11 | No (PBS) | Incubation | 1000 | 230.4 |

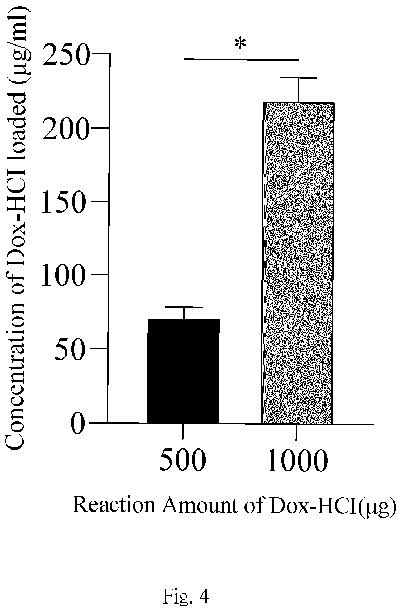

Referring to Table 3 and FIG. 4, the optimal reaction concentration of Doxorubicin hydrochloride (Dox-HCl) as the target drug was further tested. The experiments involved the drug-loading procedure described in Step 3, the reaction amount of the target drug in experiments 2-6 is at 500 µg or 1000 µg selectively. In experiments 2-6, the sonication method was used to establish the ammonium sulfate concentration gradient.

**Table 2**

| | **Exosome Particle Concentration (particles/mL)** | **Method for establish the ammonium sulfate concentration gradient** | **Target Drug Reaction Amount (µg)** | **Concentration of target drug loaded (µg/mL)** |
|---|---|---|---|---|
| **Experiment 2** | 3.3E11 | Sonication | 500 | 77.8 |
| **Experiment 3** | 3.1E11 | Sonication | 500 | 73.6 |
| **Experiment 4** | 3.1E11 | Sonication | 1000 | 230.4 |
| **Experiment 5** | 5.3E11 | Sonication | 500 | 64.5 |
| **Experiment 6** | 5.3E11 | Sonication | 1000 | 209.5 |

Referring to FIG. 4, statistical analysis was performed using an unpaired t-test to compare the results of Experiments 2, 3 and 5 with those of Experiments 4 and 6. The data revealed that drug-loading reactions using 1000 µg Dox-HCl in Experiments 4 and 6 resulted in significantly enhanced drug-loading efficiencies. Specifically, these experiments achieved almost a threefold increase in the amount of drug loaded compared to Experiments 2, 3 and 5, which used 500 µg of Dox-HCl.

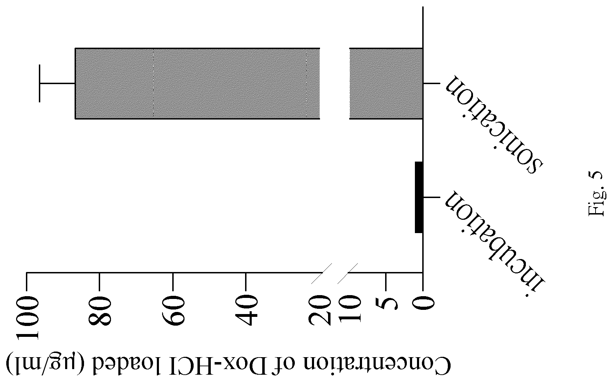

Further compare Experiments 2, 3 and 5 with the aforementioned Experiment 1 to verify whether the sonication method and the extrusion method would affect drug loading effect of exosomes 10 after establishing the ammonium sulfate concentration gradient. As shown in Figure 5, the result illustrates that using the sonication method to establish the ammonium sulfate concentration gradient, and then reacting 500 µg Dox-HCl with exosomes 10, can produce better drug loading effect (even reach nearly 70 times the amount of target drug loaded) than establishing the ammonium sulfate concentration gradient by incubation.

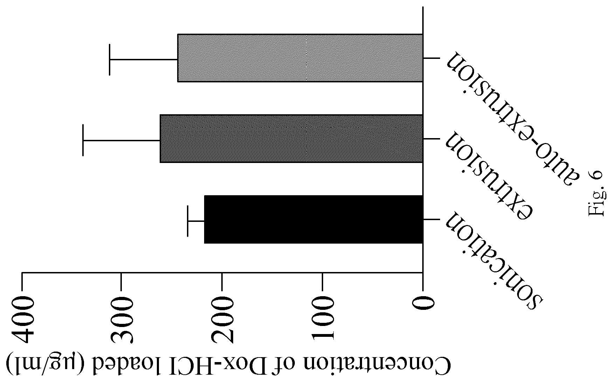

Furthermore, as shown in Table 3 and FIG. 6-7, the effect of different extrusion methods (i.e., extrusion method and auto-extrusion method) on the efficiency of drug encapsulation into the exosomes 10 was tested. The ammonium sulfate concentration gradient was established using the extrusion method for Experiments 7-12, followed by the drug-loading procedure described in Step 3, the reaction amount of the Dox-HCl is at 500 µg or 1000 µg selectively; in the Experiments 13-15, the ammonium sulfate concentration gradient was established by using the auto-extrusion method. The results obtained in Experiments 7-12 and Experiments 13-15 were compared with those obtained in Experiments 4-5 using the sonication method. In the present embodiment, the pressure provided from the extruder was between 50 and 500 psi.

**Table 3**

| | **Exosome Particle Concentration (particles/mL)** | **Method for establish the ammonium sulfate concentration gradient** | **Target Drug Reaction Amount (µg)** | **Concentration of target drug loaded (µg/mL)** |
|---|---|---|---|---|
| **Experiment 7** | 2.9E11 | extrusion | 500 | 80.9 |
| **Experiment 8** | 2.9E11 | extrusion | 500 | 93.7 |
| **Experiment 9** | 2.9E11 | extrusion | 1000 | 200.2 |
| **Experiment 10** | 3.6E11 | extrusion | 1000 | 355.9 |
| **Experiment 11** | 5.4E11 | extrusion | 1000 | 294.3 |
| **Experiment 12** | 5.4E11 | extrusion | 1000 | 209.6 |
| **Experiment 13** | 1.85E12 | auto-extrusion | 1000 | 191.8 |
| **Experiment 14** | 9.6E11 | auto-extrusion | 1000 | 232.4 |
| **Experiment 15** | 1.12E12 | auto-extrusion | 1000 | 318.7 |

Referring to FIG. 5, statistical analysis was performed using the One-way ANOVA, which revealed no significant differences in drug-loading efficiency by using the exosomes 10 between the sonication, extrusion and auto-extrusion methods for establishing the ammonium sulfate concentration gradient. Nevertheless, the extrusion and auto-extrusion method demonstrated slightly increase the overall drug-loading capacity, suggesting its potential to improve the uniformity of drug encapsulation of the exosomes 10.

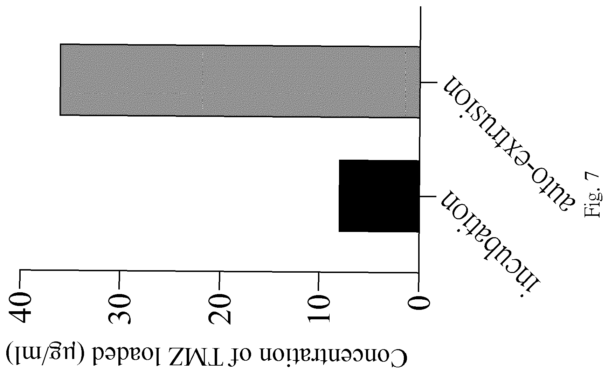

Next, please referring to Figure 7, using the TMZ as the target drug is applied to confirm the loading efficiency of the exosomes 10 after constructing the ammonium sulfate concentration gradient. Consistent with the aforementioned results, the exosomes 10 have the ability to load the target drug after constructing the ammonium sulfate concentration gradient. With the implementation of the auto-extrusion method, the exosomes 10 can be increased to nearly 3 times the drug loading capacity (the concentration of target drug loaded).

A notable aspect of the present invention is the use of a balanced crystalloid solution as the primary medium for both the exosome reaction solution 103 and the drug solution 104 during the stages of establishing the ammonium sulfate gradient in Step 2 and the drug loading in Step 3. In addition, the balanced crystalloid solution is used during the dialysis step to remove the excess drug solution 104 that is not encapsulated within the lumen 12 of the exosome 10. The application of the balanced crystalloid solution in the present invention may eliminate the need for repeated replacement of the different reaction solutions from the establishment of the ammonium sulfate concentration gradient to loading of the target drug, ensuring a stable reaction environment throughout the process, and thereby improving both the quality and efficiency of drug loaded into the exosomes 10.

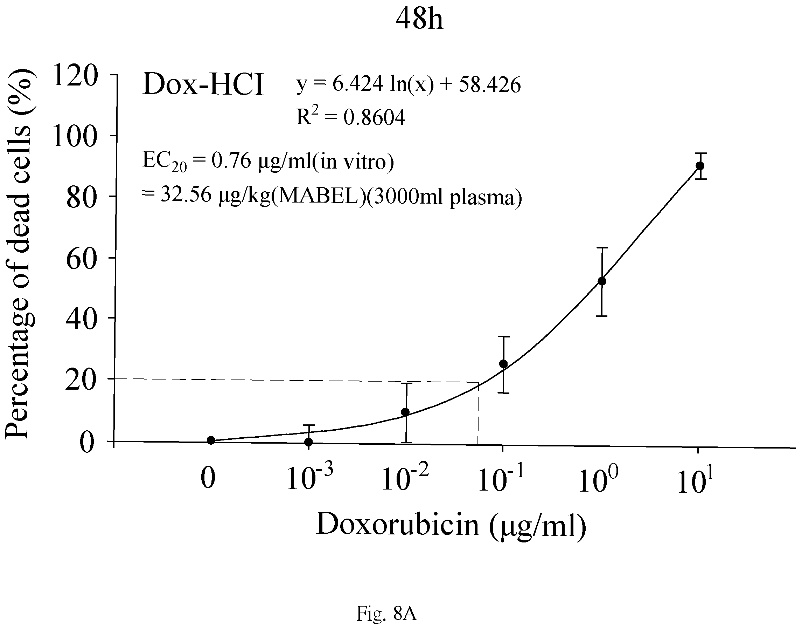

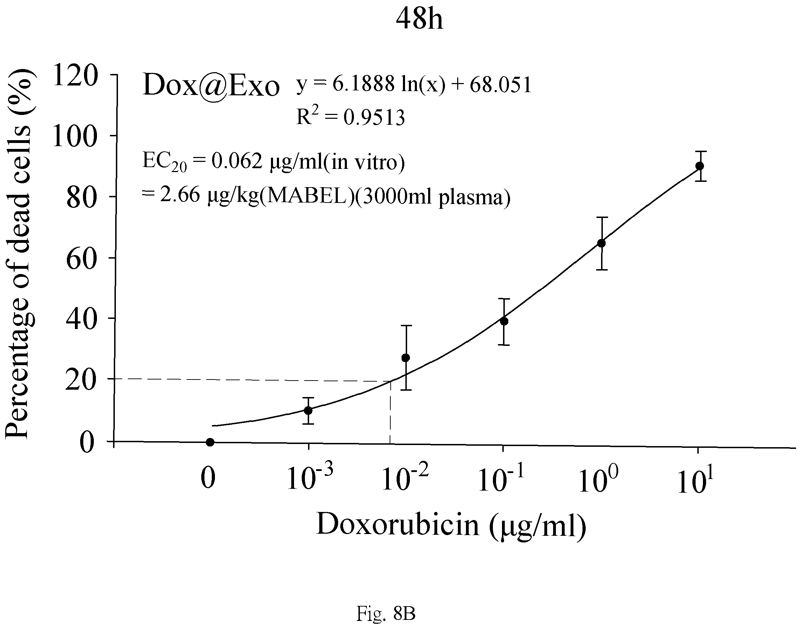

In order to verify that the exosome 10 loaded with the target drug can be endocytosed by recipient cells and can release the drug in the cells to induce a cytotoxic effect. Please refer to Figures 8A and 8B, by using a cell proliferation and cytotoxicity assay, breast cancer cells (MDA-MB-231) were co-cultured with the exosome 10 (Dox@EOX) loaded with the target drug Dox-HCl for 48 hours (as shown in Figure 8A). In this experiment, the exosome 10 is modified to express anti- human leukocyte antigen G (HLA-G) proteins on the surface before drug loading, which makes the exosome 10 exhibit a targeting capability toward HLA-G. A control group was set up by directly treating the breast cancer cells with the target drug (Dox-HCl) (as shown in Figure 8B).

The results indicate that in the control group, to achieve 20% cancer cell cytotoxicity, a required drug concentration (EC20) must reach 0.76 µg/mL when the target drug was directly incubated with breast cancer cells (Figure 8A). In contrast, the required drug concentration (EC20) was only 0.062 µg/mL when treating the cancer cells through the exosomes 10 loaded with the target drug, shown in the 8B. These results demonstrate that the exosome 10 effectively delivers the target drug to the cancer cells, significantly reducing the required drug concentration. According to the reference Schaller TH, et al. (Schaller TH, Snyder DJ, Spasojevic I, et al. First in human dose calculation of a single-chain bispecific antibody targeting glioma using the MABEL approach. Journal for ImmunoTherapy of Cancer 2020;8:e000213. doi:10.1136/jitc-2019-000213), using a standard formula provided in the reference to convert the drug doses used in the cytotoxicity assay into a human-equivalent doses. The minimum anticipated biological effect level (MABEL) for the directly incubated between the target drug with breast cancer cells was calculated as 32.56 µg/kg. However, when the exosome 10 loaded with Dox-HCl was used, the MABEL required to achieve 20% cancer cell cytotoxicity was only 2.66 µg/kg, demonstrating an over 10-fold increase in drug delivery efficiency.

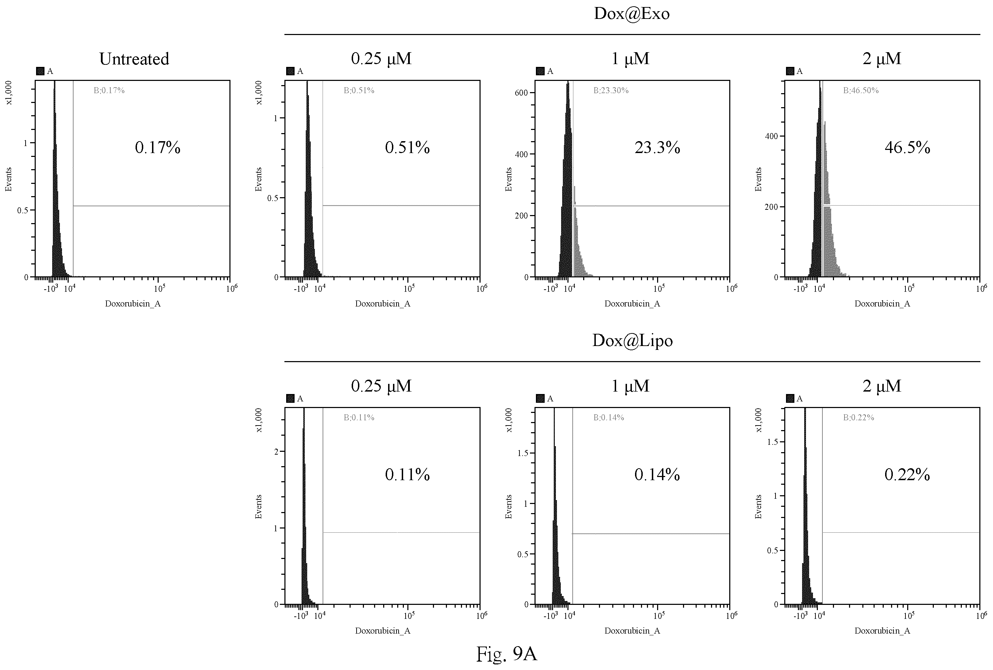

Further compare the cytotoxic effect between the exosome 10 loaded with the target drug (Dox@EOX) and a liposome injection loaded with the target drug (Dox@Lipo), which is currently available clinical drugs. Please refer to Figures 9A to 8C, during a cellular uptake assay, exosome 10 and the liposomal injection were co-cultured for 3 hours with breast cancer cells separately after loaded with Dox-HCl at concentrations of 0.25 µM, 1 µM, and 2 µM respectively. Flow cytometry was applied to detect drug signals of the target drug to evaluate the proportion of breast cancer cells which have up-taken the target drug. The results indicate that the group treated by exosome 10 loaded with Dox-HCl (Dox@EOX) exhibited a significantly higher rate of drug delivery into breast cancer cells and higher uptake proportion of the breast cancer cells compared to the group treated by the liposomal injection (Dox@Lipo).

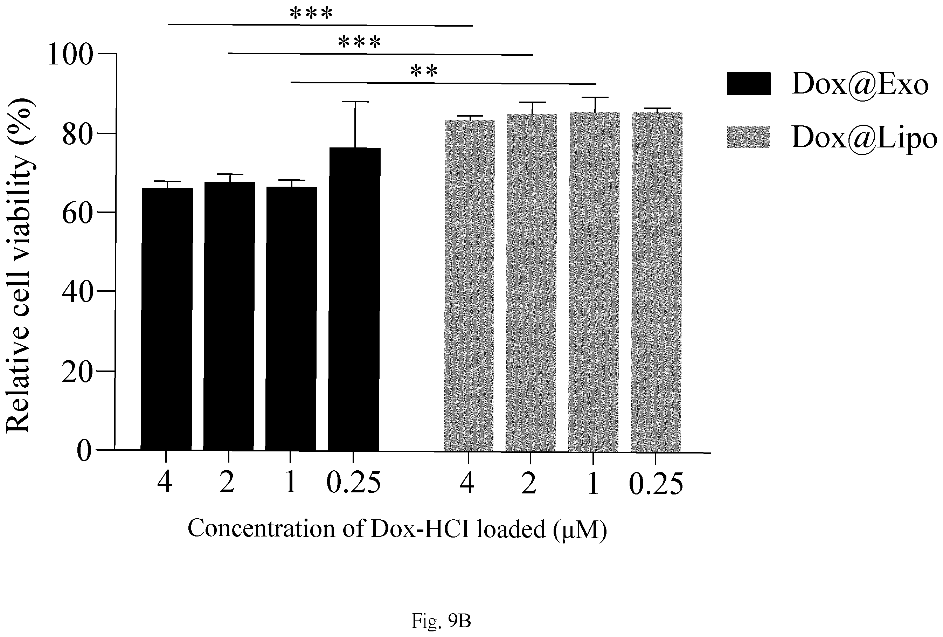

Figure 9B illustrates a cell viability of breast cancer cells after being co-cultured for 5 hours with exosome 10 loaded with the target drug Dox-HCl (Dox@EOX) and the liposomal injection loaded with Dox-HCl (Dox@Lipo). The cell viability of breast cancer cells between the cytotoxic effects of the Dox@EOX and Dox@Lipo was evaluated. After using the One-way ANOVA as the statistical analysis, exosome 10 loaded with the target drug Dox-HCl (Dox@EOX) at concentrations of 4 µM, 2 µM, and 1 µM demonstrated higher cytotoxic effect compared to the groups Dox@Lipo with the same concentration level. This indicates that drug-loaded exosome 10 can enhance the likelihood of cancer cell death within a short period, increasing the cytotoxicity rate by approximately 15% compared to the liposomal injection (Dox@Lipo).

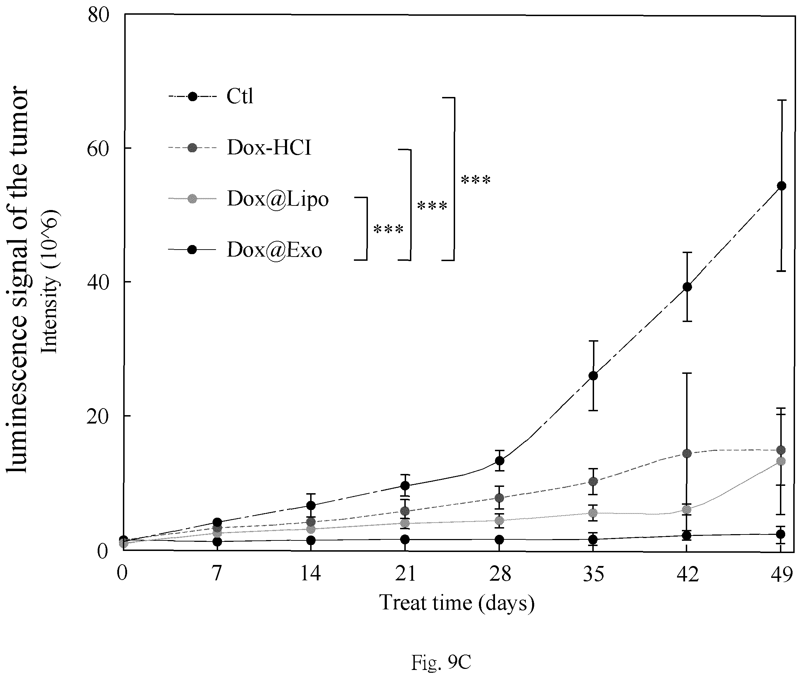

Referring to Figure 9C, a breast cancer animal model was applied to evaluate the efficacy and biocompatibility of exosome 10 loaded with the target drug. In this experiment, the breast cancer animal model was constructed by injecting the breast cancer cells (MDA-MB-231) orthotopically into the mammary fat pad of the mice and then culturing the breast cancer cells into tumor. The mice were divided into drug treatment groups 1 to 3 and control group 1. The breast cancer animal model in drug treatment groups 1 to 3 were received the target drug directly (Dox-HCl), the liposome injection loaded with the target drug Dox-HCl (Dox@Lipo), and the exosomes 10 loaded with the target drug Dox-HCl (Dox@EXO) respectively; the control group 1 is the group treated with a placebo (PBS) (Ctrl).

Each group (groups 1-3 and control group 1) of the breast cancer animal model described above was received tail vein injections continuously for six weeks administration following the injection of breast cancer cells (MDA-MB-231). Tumor development was monitored and imaged using an *in vivo* imaging system (IVIS Imaging System), which measured tumor progression based on a luminescence signal of the tumor. The results showed that two weeks after the final drug administration (on day 49), the group 3 (Dox@EXO) exhibited significantly lower tumor intensity of the luminescence signal compared to the other groups (groups 1-2) and the control group 1. Specifically, the intensity of the luminescence signal in Control Group 1 (Ctrl) was approximately 21.8 times higher than that of the Dox@EXO group. By converting the luminescence signal into a tumor volume, it can be found that inhibition rate of tumor growth in three groups (groups 1-3) were: group 1 (Dox-HCl): 73.8%; group 2 (Dox@Lipo): 76.9%, and group 3 (Dox@EXO): 97.8%. These findings demonstrate that the exosome 10 produced by the method provided in the present invention can exhibit better drug effects after being loaded with the target drug.

Furthermore, to confirm that exosome 10 retains its biocompatibility after loading the target drug and is safe for *in vivo* use, please refer to Table 5, an animal model is applied to evaluate the safety. Immunocompetent mice were divided into control group (Control), vehicle control group (Vehicle control), drug treatment group (Dox), and exosome treatment groups 1-3 (Dox@EXO 1-3). The groups above were weekly administered Lactated Ringer's solution, the exosomes 10, the target drug (Doxorubicin, Dox-HCl), and the exosomes 10 loaded with the target drug Dox-HCl (Dox@EXO) in concentrations of 2 mg/kg, 6 mg/kg, and 12 mg/kg respectively. After receiving the third administration, blood samples were collected for serum biochemical analysis to monitor organ function. Statistical analysis was performed using the One-way ANOVA, the result revealed significant differences in liver function markers such as albumin (ALB) and alkaline phosphatase (ALP) between the drug treatment group (Dox) and the control group (Control) treat with Lactated Ringer's solution. However, there were no significant differences in any serum biochemical parameters between the Dox@EXO groups 1-3 (2 mg/kg, 6 mg/kg, and 12 mg/kg) and the control group. During the experiment, all mice in the exosome treatment groups 1-3 (Dox@EXO 1-3) exhibited normal physical activity (data not shown), confirming that the exosome 10 produced from this present invention is safe for biological use.

**Table 5**

| | **control** | **Vehicle control** | **Dox (12 mg/kg)** | **Dox@EXO 1 (2 mg/kg)** | **Dox@EXO 2 (6 mg/kg)** | **Dox@EXO 3 (12 mg/kg)** |
|---|---|---|---|---|---|---|
| **AST (U/L)** | 117.74 ± 40.54 | 74.68 ± 21.35 | 76.88 ± 11.49 | 101.56 ± 53.40 | 84.58 ± 32.32 | 70.36 ± 14.33 |
| **ALT (U/L)** | 57.44 ± 25.10 | 29.74 ± 8.25 | 29.52 ± 2.58 | 44.08 ± 34.63 | 32.14 ± 5.97 | 35.12 ± 6.85 |
| **ALB (g/dL)** | 3.48 ± 0.13 | 3.36 ± 0.18 | 2.68 ± 0.45* | 3.40 ± 0.19 | 3.44 ± 0.21 | 2.98 ± 0.54 |
| **ALP (U/L)** | 312.38 ± 110.95 | 360.02 ± 16.91 | 182.52 ± 40.27* | 301.16 ± 56.61 | 256.86 ± 38.78 | 218.88 ± 45.99 |
| **LDH (U/L)** | 504.24 ± 70.50 | 487.80 ± 196.35 | 648.28 ± 212.55 | 519.06 ± 160.30 | 672.52 ± 318.21 | 570.06 ± 331.77 |
| **AMY (U/L)** | 2324.0 ± 117.3 | 2096.0 ± 133.1 | 3424.8 ± 941.0* | 2480.8 ± 419.5 | 2390.0 ± 221.5 | 3230.4 ± 1114.9 |
| **CK (U/L)** | 372.80 ± 358.45 | 206.58 ± 99.97 | 236.06 ± 66.16 | 393.96 ± 227.51 | 436.52 ± 560.48 | 177.08 ± 83.52 |

| | | | | | | |
|---|---|---|---|---|---|---|
| Abbreviations: ALB=Albumin; ALP=Alkaline Phosphatase; AMY=Amylase; CK=Creatine kinase; LDH=Lactate Dehydrogenase *: p≦0.05 | | | | | | |

A comprehensive analysis of the experimental results underscores the robustness of the disclosed method in significantly enhancing the encapsulation of one or more drugs into the exosomes 10. This approach also mitigates drug diffusion loss and reduces associated cytotoxic effects. It should be emphasized that the embodiments described herein are for the sole purpose of illustrating the technical principles and innovative features of the invention to enable those skilled in the art to implement its concepts. These embodiments should not be construed as limiting the scope of protection. For example, while Doxorubicin hydrochloride is used in the examples described, other target drugs may be substituted without departing from the essence of the invention. All equivalent modifications and adaptations derived from the principles of the present invention are intended to fall within its scope of protection.

## Claims

1. A method for optimizing drug loading efficiency into extracellular vesicles (10), designed to facilitate the encapsulation of a target drug within a lumen (12) enclosed by a lipid bilayer (11) of an extracellular vesicle (10), comprising the steps of:
preparing an extracellular vesicle concentrate (101) containing extracellular vesicles (10);
uniformly mixing the extracellular vesicle concentrate (101) with an ammonium sulfate solution (102) of an appropriate concentration, facilitating the diffusion of the ammonium sulfate solution (102) into the lumen (12) of the extracellular vesicle (10), then removing and replacing a residual ammonium sulfate solution (102) outside the lipid bilayer (11) of the extracellular vesicle (10) with a balanced crystalloid solution to form an extracellular vesicle reaction solution (103), and an ammonium sulfate concentration gradient is established between outside the lipid bilayer (11) and the lumen (12) of the extracellular vesicle (10); and
configuring a drug solution (104) by dissolving the target drug in the balanced crystalloid solution, performing a static reaction after mixing the drug solution (104) with the extracellular vesicle reaction solution (103), one part of the drug solution (104) enters the lumen (12) of the extracellular vesicle (10) through the ammonium sulfate concentration gradient during the static reaction, and subsequently removing the drug solution (104) remained outside the extracellular vesicle (10), thereby completing the encapsulation of the target drug within the extracellular vesicle (10).

2. The method according to Claim 1, wherein the balanced crystalloid solution is selected from the group consisting of a Lactated Ringer's buffer and a Plasma Lyte A (PLA) solution.

3. The method according to Claim 1, wherein the ammonium sulfate solution (102) has a concentration in the range of 0.1 M to 5 M.

4. The method according to Claim 1, wherein the ammonium sulfate solution (102) comprises an additive for assisting in constructing the ammonium sulfate concentration gradient or promoting the encapsulation of the target drug.

5. The method according to Claim 1, wherein the volume ratio of the extracellular vesicle concentrate (101) to the ammonium sulfate solution (102) is between 1:50 and 1:200.

6. The method according to Claim 1, wherein the introduction of the ammonium sulfate solution (102) into the lumen (12) is facilitated by a sonication method, adding a surfactant, or an extrusion method.

7. The method according to Claim 6, wherein the extrusion method performed via manual or auto-extrusion.

8. The method according to Claim 1, wherein the drug solution (104) contains the target drug at an effective concentration in the range of 0.5 mg/mL to 5 mg/ml.

9. The method according to Claim 1, wherein the extracellular vesicle reaction solution (103) comprises exosome particles at a concentration in the range of 10⁹ to 10¹³ particles/ml.

10. A method for enhancing the efficiency of drug loading into extracellular vesicles (10) specifically for a target drug containing an amine group, comprising the steps of:
preparing an extracellular vesicle concentrate (101) containing extracellular vesicles (10);
uniformly mixing the extracellular vesicle concentrate (101) with an ammonium sulfate solution (102) of an appropriate concentration, facilitating the diffusion of the ammonium sulfate solution (102) into the lumen (12) of the extracellular vesicle (10), then removing and replacing a residual ammonium sulfate solution (102) outside the lipid bilayer (11) of the extracellular vesicle (10) with a balanced crystalloid solution to form an extracellular vesicle reaction solution (103), and an ammonium sulfate concentration gradient is established between outside the lipid bilayer (11) and lumen (12) of the extracellular vesicle (10); and
configuring a drug solution (104) by dissolving the target drug in the balanced crystalloid solution, performing a static reaction after mixing the drug solution (104) with the extracellular vesicle reaction solution (103), one part of the drug solution (104) enters the lumen (12) of the extracellular vesicle (10) through the ammonium sulfate concentration gradient during the static reaction, and subsequently removing the drug solution (104) remained outside the extracellular vesicle (10), thereby completing the encapsulation of the target drug within the extracellular vesicle (10).

11. The method according to Claim 10, wherein the ammonium sulfate solution (102) comprises an additive for assisting in constructing the ammonium sulfate concentration gradient or promoting the encapsulation of the target drug.

12. The method according to Claim 10, wherein the drug solution (104) contains the target drug at a reaction amount in the range of from 500 µg to 1000 µg, and the extracellular vesicle reaction solution (103) comprises exosome particles at a concentration in the range of from 10⁹ to 10¹³ particles/ml.

13. The method according to Claim 10, wherein the balanced crystalloid solution is selected from the group consisting of a Lactated Ringer's buffer and a Plasma Lyte A (PLA) solution.

14. The method according to Claim 10, wherein the ammonium sulfate solution (102) has a concentration in the range of 0.1 M to 5 M.

15. The method according to Claim 10, wherein the volume ratio of the extracellular vesicle concentrate (101) to the ammonium sulfate solution (102) is between 1:50 and 1:200.

16. The method according to Claim 10, wherein the target drug comprising Doxorubicin hydrochloride (Dox-HCl) or Temozolomide (TMZ).

17. A medicinal product, produced by a method provided in Claim 1.

18. A medicinal product, produced by a method provided in Claim 10.

19. The medicinal product according to Claim 18, including a target drug, which is Doxorubicin hydrochloride (Dox-HCl) or Temozolomide (TMZ).
